(19) Europäisches Patentamt
European Patent Office
Office européen des brevets

(11) Veröffentlichungsnummer : **0 112 538 B1**

(12) # EUROPÄISCHE PATENTSCHRIFT

(45) Veröffentlichungstag der Patentschrift : **21.10.87**

(51) Int. Cl.⁴ : **C 07 D475/02**

(21) Anmeldenummer : **83112571.1**

(22) Anmeldetag : **14.12.83**

(54) **Verbessertes Verfahren zum Reinigen von Riboflavin.**

(30) Priorität : **22.12.82 DE 3247381**

(43) Veröffentlichungstag der Anmeldung : **04.07.84 Patentblatt 84/27**

(45) Bekanntmachung des Hinweises auf die Patenterteilung : **21.10.87 Patentblatt 87/43**

(84) Benannte Vertragsstaaten : **CH DE FR GB LI NL**

(56) Entgegenhaltungen : **US-A- 2 324 800**

(73) Patentinhaber : **BASF Aktiengesellschaft**
**Carl-Bosch-Strasse 38**
**D-6700 Ludwigshafen (DE)**

(72) Erfinder : **Grimmer, Johannes**
**Aeblerose Vej 11**
**DK-8500 Grehaa (DK)**
Erfinder : **Horn, Hans Christoph, Dr.**
**Roemerstrasse 22**
**D-6715 Lambsheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 112 538 B1

Jouve, 18, rue St-Denis, 75001 Paris, France

**Beschreibung**

Die vorliegende Erfindung betrifft ein verbessertes Verfahren zur Hochreinigung von rohem Riboflavin (Vitamin $B_2$) durch oxidative Behandlung des rohen Riboflavins in mineralsaurer Lösung, vorzugsweise in salpetersaurer Lösung.

Riboflavin (I) wird üblicherweise, wie allgemein bekannt ist (vgl. z. B. W. H. Sebrell et R. S. Harris, « The Vitamins ; Chemistry, Physiologie, Pathology, Methods », 2. Auflage, Band V, 1972, Academic Press, Seite 22) durch Kondensation eines N-(D)-Ribityl-2-arylazo-4,5-dimethylanilins (II) mit Barbitursäure (III) synthetisch hergestellt.

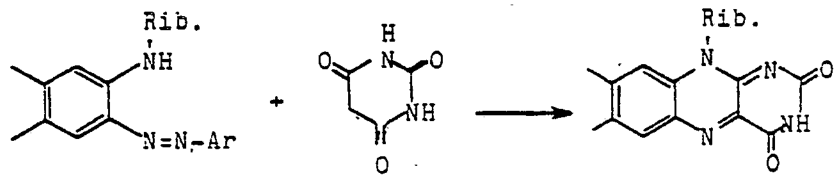

(II) (III) (I)

Rib = D-Ribityl
Ar = Aryl, z. B. Phenyl

Hierbei erhält man ein Rohprodukt, welches neben etwa 92 bis 96 Gew.% I noch verschiedene Verunreinigungen enthält, darunter beispielsweise Barbitursäure, Dibarbitursäure, Lumiflavin und Lumichrom.

Auch die Kondensation anderer N-(D)-Ribityl-4,5-dimethyl-anilinderivate mit Barbitursäurederivaten zu Riboflavin wurde schon beschrieben.

Da sich reines Riboflavin durch Kristallisationsvorgänge allein nur mit unverhältnismäßig hohem Aufwand aus der Rohware gewinnen läßt, unterwirft man das rohe Riboflavin nach der Lehre der US-PS 2 324 800 einer oxidativen Behandlung in wäßrig-saurem Milieu, wonach man eventuelle Niederschläge abtrennt und die verbleibende Lösung mit viel Wasser verdünnt. Hierbei fällt das Riboflavin in Form von gelben Nadeln an, die nur noch abfiltriert und gewaschen zu werden brauchen.

Gemäß der genannten US-PS löst man das rohe Riboflavin in einer mit nicht mehr als zwei Volumenteilen Wasser verdünnten nichtoxidativen Mineralsäure, wie Salzsäure, Schwefelsäure oder ortho-Phosphorsäure auf, oxidiert die Verunreinigungen durch Behandlung der mineralsauren Lösung mit Oxidationsmitteln, wie Chlor, Wasserstoffperoxid oder Chlorsäure, filtriert ausgeschiedene unlösliche Produkte ab und gießt die Reaktionslösung zwecks Abscheidung des Riboflavins in einen Überschuß an Wasser. Auch die Verwendung von wäßriger Salpetesäure als gleichzeitig saurem und oxidativen Agens wurde in der genannten US-PS schon vorgeschlagen. Nachteilig an dem bekannten Verfahren sind die relativ langen Reaktionszeiten sowie der ungenügende Reinheitsgrad des erhaltenen Riboflavin-Niederschlags. So erfolgte beispielsweise die Reinigung des Riboflavins mit 46 gew.%iger Salpetersäure bei 50 bis 60 °C im Verlauf von 3 Stunden. Anschließend wurden ausgeschiedene Verunreinigungen abfiltriert und die klare Lösung in 75 °C heißes Wasser gegeben, wobei ein amorpher Niederschlag erhalten wurde. Es war daher erforderlich, die wäßrige Suspension zwecks Gewinnung einer reinen kristallinen Masse noch einige Stunden bei 90 °C zu rühren. Abgesehen davon, daß hierbei noch etwa 15 % des eingesetzten Riboflavins verlorengehen, ist diese Arbeitsweise zeitraubend und technisch umständlich. Ähnlich verhält es sich mit den übrigen Ausführungsformen der zitierten US-PS, z. B. bei Verwendung von Salzsäure und Wasserstoffperoxid oder Chlor.

Weiterhin ist aus der JA-OS 10151/1968 ein Verfahren zur Herstellung von Riboflavin durch Umsetzen von II mit III bekannt, bei dem das erhaltene Rohriboflavin durch Lösen in verdünnter Salzsäure, Zugabe von Wasserstoffperoxidlösung, anschließendes Erhitzen, Abfiltrieren und Eingießen in warmes Wasser gereinigt wird. Nachteilig an diesem Verfahren ist, daß dem erhaltenen Riboflavin trotz Reinigung noch ein unangenehmer Geruch anhaftet, der sich bei der Verwendung des Vitamins, insbesondere in der menschlichen Ernährung, störend bemerkbar macht.

Der Erfindung lag daher die Aufgabe zugrunde, die geschilderten Nachteile durch Verbesserung des oxidativen Verfahrens zur Reinigung von rohem Riboflavin zu beseitigen.

Gegenstand der Erfindung ist ein verbessertes Verfahren zur Reinigung von rohem Riboflavin, welches durch Kondensation eines N-(D)-Ribityl-2-arylazo-4,5-dimethyl-anilins und Barbitursäure oder eines anderen N-(D)-Ribityl-4,5-dimethyl-anilinderivates mit einem Barbitursäurederivat hergestellt wurde, durch Lösen des rohen Riboflavins in mit Wasser verdünnter Schwefelsäure oder Phosphorsäure und Oxidieren der Verunreinigungen durch Behandeln der Lösung mit Wasserstoffperoxid oder durch Lösen in verdünnter Salpetersäure bei erhöhter Temperatur und anschließende Gewinnung des reinen Riboflavins durch Ausfällen mit Wasser, das dadurch gekennzeichnet ist, daß man die mineralsaure Lösung des Riboflavins und des Oxidationsmittels bzw. die Lösung des Riboflavins in 20 bis 70 gew.-

%iger Salpetersäure möglichst rasch auf eine Temperatur erhitzt, bei der ein deutlicher Farbumschlag der Lösung von grünlich nach gelb-orange zu beobachten ist, die Lösung 1 bis 100, vorzugsweise 1 bis 50, insbesondere 1 bis 20 Sekunden bei dieser Temperatur hält und danach die Oxidationsreaktion durch Zugabe von Wasser abbricht.

Überraschenderweise gelingt das erfindungsgemäße Verfahren besonders vorteilhaft, wenn man eine Lösung von rohem Riboflavin in 20 bis 70 gew.%iger wäßriger Salpetersäure möglichst rasch auf die Temperatur erhitzt, bei der ein deutlicher Farbumschlag der Lösung von grünlich nach gelb-orange zu beobachten ist.

Weiterhin wurde gefunden, daß man bei dem erfindungsgemäßen Verfahren die besten Ergebnisse erzielt, wenn man das Reaktionsgemisch sofort oder nach vorherigem Abbruch der Oxidationsreaktion durch Zugabe von kaltem Wasser in 90 bis 100 °C heißes Wasser leitet und die sich hierbei bildende Suspension noch etwa 10 bis 30 Minuten bei dieser Temperatur hält.

Das erfindungsgemäße Verfahren beruht auf der überraschenden Beobachtung, daß die oxidative Zerstörung der Verunreinigungen innerhalb weniger Sekunden in einem eng begrenzten Temperaturintervall stattfindet, ohne daß das empfindliche Riboflavin selber nennenswert angegriffen wird, wie dies bei den vorbekannten Methoden geschieht.

Ganz besonders vorteilhaft gestaltet sich das erfindungsgemäße Verfahren bei Verwendung von wäßriger Salpetersäure als gleichzeitig saurem und oxidativem Reagenz. Abgesehen davon, daß sich das Verfahren mit Salpetersäure technisch leichter realisieren läßt, erzielt man hiermit etwas bessere Ausbeuten und ein etwas reineres Riboflavin. Überraschenderweise erhält man hiermit ein reines und völlig geruchloses Produkt, so daß man auf ein Waschen des Endproduktes mit Methanol verzichten kann, was beispielsweise bei der Verwendung von wäßriger Salzsäure und $Cl_2$ oder $H_2O_2$ unumgänglich ist.

Demgegenüber werden gemäß US 2 324 800 insbesondere die Verwendung von Salzsäure und $Cl_2$ oder Salzsäure mit einem anderen oxidierenden Agens, das bei der Wechselwirkung mit Salzsäure $Cl_2$ ergibt, empfohlen.

Wir werden das erfindungsgemäße Verfahren anhand seiner bevorzugten Ausführungsform, d. h. der Arbeitsweise mit wäßriger Salpetersäure näher erläutern. Bezüglich Art und Konzentration der übrigen Mineralsäuren und der Oxidationsmittel gelten die Bedingungen der angegebenen US-Patentschrift.

Die Oxidationstemperatur, also die Temperatur des Farbumschlags, der außerdem von einem heftigen Schäumen begleitet wird, ist in gewissem Grade abhängig von Art und Konzentration der Säure und des Oxidationsmittels. Bei der Verwendung von wäßriger Salpetersäure als saurem und oxidativem Agens ist sie eine Funktion der Salpetersäurekonzentration. Je geringer diese ist, um so höher ist die Oxidationstemperatur und umgekehrt.

Die folgende Tabelle veranschaulicht diesen Zusammenhang, wobei im Einzelfall je nach Provenienz des rohen Riboflavins jedoch Abweichungen auftreten können.

| Salpetersäurekonzentration Gew.% | Temperatur des Farbumschlages °C |
|---|---|
| 20 | 98 |
| 30 | 94 |
| 40 | 87 |
| 46 | 84 |
| 50 | 78 |
| 62,5 | 72 |

Geringere $HNO_3$-Konzentrationen als 20 Gew.% wird man in der Regel nicht wählen, damit man nicht zu hoch erhitzen muß, und höhere Konzentrationen als 70 Gew.% sind im allgemeinen weniger zweckmäßig, weil teilweise eine Zerstörung des Riboflavinmoleküls erfolgt.

Lediglich beim Arbeiten in einem geschlossenen Reaktionsgefäß unter Druck könnten Salpetersäurekonzentrationen kleiner als 20 Gew.% und höhere Temperaturen als 100 °C angewandt werden.

Die Menge an Salpetersäure bzw. die Konzentration von Riboflavin in der wäßrigen Salpetersäure kann in weiten Grenzen variiert werden. Es ist natürlich am wirtschaftlichsten, mit möglichst wenig $HNO_3$ zu arbeiten. Bei Verwendung von zu wenig $HNO_3$ werden keine Lösungen mehr gebildet, sondern Suspensionen. Mit Vorteil arbeitet man mit etwa 1,5 bis 3 Gew.-Teilen der wäßrigen Salpetersäure pro Gew.-Teil Riboflavin.

Weiterhin richtet sich die Menge der wäßrigen $HNO_3$ nach deren Konzentration. Liegt sie unter 10 Gew.%, nimmt die Löslichkeit für das Riboflavin stark ab.

Die beste Löslichkeit hat das rohe Riboflavin in Salpetersäure von Konzentrationen zwischen 30 und 65 Gew.%. Hier benötigt man etwa 0,9 bis 2 l der Säure, um 1 kg des rohen Riboflavins bei Raumtemperatur zur Lösung zu bringen. Entsprechend weniger Säure ist erforderlich, wenn man das Riboflavin bei höheren Temperaturen löst ; jedoch sollte es etwa 20 °C unterhalb der Farbumschlagstemperatur bereits vollständig in Lösung vorliegen.

Bei der Farbumschlagstemperatur findet offensichtlich eine spontane oxidative Zersetzung der störenden Begleitstoffe statt, ohne daß das Riboflavin dabei nennenswert angegriffen wird. Sowie diese

Temperatur erreicht wird, sollte die Reaktion möglichst schnell abgebrochen werden, damit die Oxidation nicht auf das Riboflavin übergreift.

Der Abbruch der Reaktion erfolgt durch rasches Abkühlen und/oder durch Herabsetzen des Oxidationspotentials der Lösung, indem man die Lösung mit Wasser verdünnt. Hierzu versetzt man die Reaktionslösung möglichst schnell mit etwa 25 bis 40 Gew.% der Lösung an kaltem Wasser.

Die weitere Aufarbeitung der abgebremsten Reaktionslösung erfolgt mit Vorteil so, daß man diese Lösung in einem grossen Überschuß an kochendheißem Wasser einträgt. Hierbei fällt das Riboflavin zunächst in amorpher oder amorph erscheinender Form aus, wandelt sich aber im Laufe von etwa 10 bis 30 Minuten in eine kristalline Masse um.

Die Menge an kochendem Wasser beträgt zweckmäßigerweise etwa 4 bis 10 l pro Liter der salpetersauren Lösung.

Wenn sich die Kristalle gebildet haben, was deutlich zu erkennen ist, läßt man das Gemisch abkühlen, wonach man die kristalline Masse abtrennt und mit Wasser wäscht.

Prinzipiell kann man jedoch die erfindungsgemäße Umsetzung so durchführen, daß man die Reaktionslösung nach dem Farbumschlag von grünlich nach gelb-orange dadurch abbricht, daß man sie gleich in einen Überschuß an Wasser von 90 bis 100 °C einträgt.

Mit besonderem Vorteil läßt sich das erfindungsgemäße Reinigungsverfahren kontinuierlich durchführen. Bei der kontinuierlichen Arbeitsweise ist es besonders zweckmäßig, die mineralsaure, mit dem Oxidationsmittel versetzte Lösung bzw. die salpetersaure Lösung des rohen Riboflavins mit hoher Geschwindigkeit durch ein beheiztes dünnes Rohr zu leiten, das auf einer Länge, welche einer Verweilzeit von etwa 1 bis 50 sec entspricht, auf der Farbumschlagstemperatur gehalten wird. Unmittelbar danach leitet man das Reaktionsgemisch in kochendes Wasser. Ist die Wegstrecke zu lang, kann man die Oxidation auch durch Zudosieren von kaltem Wasser schon im Rohr zum Stillstand bringen.

Die Farbumschlagstemperatur ist für eine bestimmte Riboflavinqualität und bei gleichbleibender Oxidationslösung bzw. bei einer bestimmten Salpetersäurekonzentration nach den bisherigen Beobachtungen konstant, so daß es genügt, diese Temperatur im Vorversuch zu ermitteln. Im praktischen Betrieb braucht dann nur noch diese Temperatur oder das um diese Temperatur liegende definitionsgemäße Temperaturintervall eingehalten zu werden, ohne daß es einer weiteren Überwachung und Reaktionssteuerung bedarf.

Nach dem erfindungsgemäßen Verfahren erhält man das 100 %ig reine Riboflavin in Pharmaqualität in Form eines völlig geruchlosen dottergelben Pulvers und in einer Ausbeute von 94 bis 98 % der Theorie, bezogen auf den Riboflavingehalt des Rohproduktes.

## Beispiel 1

In einer Mischung aus 50 ml einer 62,5 %igen Salpetersäure und 40 ml Wasser wurden bei 60 °C 40 g Riboflavin mit einem Gehalt von 92,7 % Riboflavin gelöst und die Lösung rasch auf 90 bis 92 °C erhitzt. Nach Aufschäumen der Reaktionslösung und einem Farbumschlag von grün nach gelb-orange wurden sofort 40 ml kaltes Wasser zugefügt. Anschließend wurde die Lösung in 800 ml siedend heißes Wassers eingetragen. Innerhalb von 20 Minuten erfolgte bei dieser Temperatur eine Kristallumwandlung. Nach Abkühlen auf 30 °C wurde abgesaugt und der Rückstand mit insgesamt 225 ml Wasser gewaschen. Nach dem Trocknen erhielt man 36,2 g gelbfluoreszierende Kristalle mit einem Gehalt an Riboflavin von 100 %, was einer Ausbeute von 97,8 % der Theorie, bezogen auf den Riboflavingehalt des Rohprodukts, entspricht.

## Beispiel 2

Durch ein U-förmig gebogenes Glasrohr (Innendurchmesser 4 mm), welches über eine Länge von 30 cm mittels eines 120 °C heißen Heizbades erhitzt wurde, wurden pro Minute 11 ml einer Lösung von 3,49 g rohem Riboflavin (92,7 %ige Reinheit) in 9,5 ml einer 60 °C heißen 40 gew.%igen Salpetersäure gepumpt. Die Verweilzeit der Lösung in der erhitzten Strecke, bei welcher die durch den Farbumschlag von grün nach gelb-orange gekennzeichnete Oxidationsreaktion stattfand, betrug 20,6 sec. Unmittelbar nach der beheizten Strecke wurde die Lösung über ein T-Stück mit 4 ml/min Wasser versetzt und dann einem Mischgefäß zugeführt, das gleichzeitig mit 70 ml/min kochendem Wasser (95 bis 98 °C) beschickt wurde. Die Suspension, die sich hierbei bildete, wurde mit einer mittleren Verweilzeit von 15 min bei 95 bis 98 °C unter Rühren in diesem Gefäß belassen, bevor sie kontinuierlich ausgetragen wurde.

Nach Abkühlen auf 30 °C, Abfiltrieren, Waschen mit Wasser und Trocknen erhielt man 185,3 g Riboflavin/Stunde einer Reinheit von 100 %. Das entspricht einer Ausbeute von 95,4 % der Theorie, bezogen auf den Riboflavingehalt des Rohproduktes.

**Patentansprüche**

1. Verbessertes Verfahren zur Reinigung von rohem Riboflavin, welches durch Kondensation eines N-(D)-Ribityl-2-arylazo-4,5-dimethyl-anilins und Barbitursäure oder eines anderen N-(D)-Ribityl-4,5-dimethyl-anilin-derivates mit einem Barbitursäurederivat hergestellt wurde, durch Lösen des rohen Riboflavins in mit Wasser verdünnter Schwefelsäure oder Phosphorsäure und Oxidieren der Verunreinigungen durch Behandeln der Lösung mit Wasserstoffperoxid oder durch Lösen in verdünnter Salpetersäure bei erhöhter Temperatur und anschließende Gewinnung des reinen Riboflavins durch Ausfällen mit Wasser, dadurch gekennzeichnet, daß man die mineralsaure Lösung des Riboflavins und des Oxidationsmittels bzw. die Lösung des Riboflavins in 20 bis 70 gew.-%iger Salpetersäure möglichst rasch auf eine Temperatur erhitzt, bei der ein deutlicher Farbumschlag der Lösung von grünlich nach gelb-orange zu beobachten ist, die Lösung 1 bis 100, insbesondere 1 bis 50 Sekunden bei dieser Temperatur hält und danach die Oxidationsreaktion durch Zugabe von Wasser abbricht.

2. Verfahren zur Reinigung von rohem Riboflavin gemäß Anspruch 1, dadurch gekennzeichnet, daß man eine Lösung von rohem Riboflavin in 20 bis 70 gew.%iger Salpetersäure möglichst rasch auf die Temperatur erhitzt, bei der ein deutlicher Farbumschlag der Lösung von grünlich nach gelb-orange zu beobachten ist.

3. Verfahren zur Reinigung von rohem Riboflavin gemäß Anspruch 1, dadurch gekennzeichnet, daß man die salpetersaure Lösung nach dem Farbumschlag sofort oder nach vorherigem Abbruch der Oxidationsreaktion durch Zugabe von kaltem Wasser in 90 bis 100 °C heißes Wasser leitet und die sich bildende Suspension noch etwa 10 bis 30 min bei dieser Temperatur hält.

4. Verbessertes Verfahren zur Reinigung von rohem Riboflavin, welches durch Kondensation eines N-(D)-Ribityl-2-arylazo-4,5-dimethyl-anilins und Barbitursäure hergestellt wurde, durch Lösen und Behandeln des rohen Riboflavins in wäßriger Salpetersäure bei erhöhter Temperatur und anschließende Gewinnung des reinen Riboflavins durch Ausfällen mit Wasser, dadurch gekennzeichnet, daß man die salpetersaure Lösung möglichst rasch auf eine Temperatur erhitzt, bei der ein deutlicher Farbumschlag der Lösung von grünlich nach gelb-orange zu beobachten ist, und daß man die salpetersaure Lösung nach dem Farbumschlag sofort oder nach vorherigem Abbruch der Oxidationsreaktion durch Zugabe von kaltem Wasser in 90 bis 100 °C heißes Wasser leitet und die sich bildende Suspension noch etwa 10 bis 30 min bei dieser Temperatur hält.

## Claims

1. An improved process for the purification of crude riboflavin which has been prepared by condensation of an N-(D)-ribityl-2-arylazo-4,5-dimethylaniline and barbituric acid or another N-(D)-ribityl-4,5-dimethylaniline derivative with a barbituric acid derivative, by dissolving the crude riboflavin in sulphuric acid or phosphoric acid which has been diluted with water, and oxidizing the impurities by treating the solution with hydrogen peroxide, or by dissolving the riboflavin in dilute nitric acid at elevated temperature and then isolating the pure riboflavin by precipitation with water, wherein the mineral acid solution of the riboflavin and the oxidizing agent, or the solution of the riboflavin in 20 to 70 % strength by weight nitric acid, is heated as rapidly as possible to a temperature at which there is a distinct change in the colour of the solution from a greenish to a yellowish orange shade, the solution is kept at this temperature for from 1 to 100, in particular from 1 to 50, seconds and the oxidation reaction is then interrupted by the addition of water.

2. A process for the purification of crude riboflavin as claimed in claim 1, wherein a solution of crude riboflavin in from 20 to 70 % strength by weight nitric acid is heated as rapidly as possible to the temperature at which there is a distinct change in the colour of the solution from a greenish to a yellowish orange shade.

3. A process for the purification of crude riboflavin as claimed in claim 1, wherein, after the change in colour, the nitric acid solution is passed, immediately or after prior interruption of the oxidation reaction by the addition of cold water, into hot water at from 90 to 100 °C, and the suspension formed is kept at this temperature for about a further 10 to 30 minutes.

4. An improved process for the purification of crude riboflavin which has been prepared by condensation of an N-(D)-ribityl-2-arylazo-4,5-dimethylaniline and barbituric acid, by dissolving and treating the crude riboflavin with aqueous nitric acid at elevated temperature and then isolating the pure riboflavin by precipitation with water, wherein the nitric acid solution is heated as rapidly as possible to a temperature at which there is a distinct change in the colour of the solution from a greenish to a yellowish orange shade, and, after the change in colour, the nitric acid solution is passed, immediately or after prior interruption of the oxidation reaction by the addition of cold water, into hot water at from 90 to 100 °C, and the suspension which forms is kept at this temperature for about a further 10 to 30 minutes.

## Revendications

1. Procédé amélioré de purification de riboflavine brute, qui a été préparée par condensation d'une N-(D)-ribityl-2-arylazo-4,5-diméthyl-aniline avec l'acide barbiturique ou d'un autre dérivé de N-(D)-ribityl-4,5-diméthyl-aniline avec un dérivé de l'acide barbiturique, par dissolution de la riboflavine brute dans de l'acide sulfurique ou de l'acide phosphorique dilué avec de l'eau et oxydation des impuretés par

traitement de la solution avec du peroxyde d'hydrogène ou par dissolution dans de l'acide nitrique dilué à température élevée, et récupération subséquente de la riboflavine pure par précipitation au moyen d'eau, caractérisé en ce que l'on chauffe le plus vite possible la solution dans un acide minéral de la riboflavine et de l'agent d'oxydation ou bien la solution de la riboflavine dans de l'acide nitrique à 20-70 % en poids à une température à laquelle on observe un changement net de couleur de la solution, qui passe d'une teinte verdâtre à une teinte jaune-orange, en ce que l'on maintient la solution à cette température pendant 1 à 100, en particulier 1 à 50 secondes, et en ce que l'on stoppe ensuite la réaction d'oxydation par une addition d'eau.

2. Procédé de purification de riboflavine brute suivant la revendication 1, caractérisé en ce que l'on chauffe le plus vite possible une solution de riboflavine brute dans de l'acide nitrique à 20-70 % en poids à la température à laquelle on observe un changement net de couleur de la solution, qui passe d'une teinte verdâtre à une teinte jaune-orange.

3. Procédé de purification de riboflavine brute suivant la revendication 1, caractérisé en ce que l'on fait passer dans de l'eau chaude à une température de 90 à 100 °C la solution dans l'acide nitrique après le changement de couleur, soit immédiatement soit après avoir stoppé au préalable la réaction d'oxydation par addition d'eau froide, et en ce que l'on maintient la suspension qui se forme pendant encore environ 10 à 30 minutes à ladite température de 90 à 100 °C.

4. Procédé amélioré de purification de riboflavine brute, qui a été préparée par condensation d'une N-(D)-ribityl-2-arylazo-4,5-diméthyl-aniline avec de l'acide barbiturique, par dissolution et traitement de la riboflavine brute dans de l'acide nitrique aqueux à température élevée et récupération subséquente de la riboflavine pure par précipitation au moyen d'eau, caractérisé en ce que l'on chauffe le plus vite possible la solution dans l'acide nitrique à une température à laquelle on observe un changement net de couleur de la solution qui passe d'une teinte verdâtre à une teinte jaune-orange, et en ce que l'on fait passer la solution dans l'acide nitrique dans de l'eau chaude à une température de 90 à 100 °C, après le changement de couleur, soit immédiatement soit après avoir stoppé au préalable la réaction d'oxydation par une addition d'eau froide, et en ce que l'on maintient la suspension qui se forme pendant encore environ 10 à 30 minutes à ladite température de 90 à 100 °C.